# EUROPÄISCHE PATENTANMELDUNG

(11) **EP 3 015 553 A1**
(43) Veröffentlichungstag der Anmeldung: **04.05.2016**
(21) Anmeldenummer: 14191173.5
(22) Anmeldetag: 30.10.2014
(51) Int. Cl.: C12Q 1/68

(54) **Stabilisierte Reaktionsmischung**

(71) Anmelder: Biotecon Diagnostics GmbH, 14473 Postdam (DE)
(72) Erfinder: Giese, Matthias, 06861 Dessau (DE); Grönewald, Cord, 13353 Berlin (DE); Berghof-Jäger, Kornelia, 12355 Berlin (DE)
(74) Vertreter: Fabry, Bernd

(57) **Zusammenfassung**

Vorgeschlagen wird eine Reagensmischungen, enthaltend
(a) eine PCR-Grundmischung, und
(b) einen kohlenhydrathaltigen Schutzstoff, umfassend oder bestehend aus
(b1) mindestens einen Stabilisator aus der Gruppe der Disaccharide und/oder Trisaccharide,
(b2) mindestens einen Gerüststoff aus der Gruppe der Dextrane, Dextrine oder Proteine, sowie gegebenenfalls
(b3) mindestens einem oberflächenaktiven Stoff.

## Beschreibung

### GEBIET DER ERFINDUNG

Die Erfindung befindet sich auf dem Gebiet der Diagnostika und betrifft Reagensmischungen für die PCR in lyophilisierter Form, die durch die Mitverwendung von kohlenhydrathaltigen Schutzverbindungen während der Gefriertrocknung vor Schädigungen geschützt werden, entsprechende Schutzverbindungen, eine Verfahren zur Gefriertrocknung von Reagensmischungen sowie die Verwendung der kohlenhydrathaltigen Zubereitungen.

### STAND DER TECHNIK

Die Polymerase-Kettenreaktion (PCR) stellt ein Verfahren dar, mit dessen Hilfe man DNA-Sequenzen mit Hilfe des Enzyms Polymerase durch wiederholte Verdoppelung in mehreren Zyklen vervielfältigt.

PCR wird eingesetzt, um einen kurzen, genau definierten Teil eines DNA-Strangs zu vervielfältigen. Der zu vervielfältigende Bereich der DNA wird als *Amplicon* bezeichnet, die bei der PCR entstehenden Produkte als *Amplifikate.* Dabei kann es sich um ein Gen oder auch nur um einen Teil eines Gens handeln oder auch um nichtcodierende DNA-Sequenzen. Im Gegensatz zu lebenden Organismen kann der PCR-Prozess nur relativ kurze DNA-Abschnitte kopieren. Bei einer Standard-PCR können dies bis zu etwa dreitausend Basenpaare (3 kbp) lange DNA-Fragmente sein. Mithilfe bestimmter Polymerasen-Gemische, weiterer Additive in der PCR und optimalen Bedingungen können sogar Fragmente mit einer Länge von über 20-40 kbp vervielfältigt werden,

Heute dient die PCR zu unterschiedlichsten Zwecken, wie beispielsweise:
- Herstellung genetischer Fingerabdrücke;
- Erkennung von Krankheiten;
- Nachweis von Pathogenen und anderen Keimen;
- Routineuntersuchungen von Blutkonserven;
- Vaterschaftstests;
- Klonierung von Genen für die medizinische Forschung und vieles mehr.

Von einer Vielzahl von Herstellern diagnostischer Zubereitungen werden heute fertige Reagensmischungen angeboten, die bereits alle Bestandteile enthalten, die für die Durchführung einer PCR, speziell einer Echtzeit-PCR benötigt werden. Diese Kits enthalten die Komponenten in wasserfreier Form, so dass diese nur noch resuspendiert werden müssen. Das Verfahren der Wahl zur Herstellung dieser Pulver besteht darin, lösungsmittelhaltige, speziell wässrige Vorgemische einer Gefriertrocknung zu unterwerfen. Die so erhaltenen Produkte weisen in der Regel weniger als 1 Gew.-% Restwasser auf und sind dadurch über längere Zeit lagerstabil.

Problematisch ist jedoch, dass die Lyophilisierung zwar ein an sich schonendes Trocknungsverfahren darstellt, jedoch unter Umständen doch dazu führen kann, dass den besonders empfindlichen Komponenten der Reagensmischung - Zellen, Enzyme, Nukleotide - Schaden zugefügt wird, was zu einer vollständigen oder teilweisen Deaktivierung führen kann.

Das Problem ist aus dem Stand der Technik hinreichend bekannt. Zum Schutz empfindlicher Reagenzien werden daher so genannte Schutzverbindungen zugegeben, bei denen es sich beispielsweise um Kohlenhydrate, wie etwa Mannitol handelt **(**DE 4305225 A1**,** ALTENA). Die Praxis zeigt jedoch, dass Schutzverbindungen der bisher bekannten Art gerade nicht in der Lage sind besonders empfindliche Reagenzien vor Schädigung zu bewahren.

Die Aufgabe der vorliegenden Erfindung hat daher darin bestanden, Reagensmischungen zur Verfügung zu stellen, die gegen Schädigungen bei der Gefriertrocknung besonders stabilisiert sind sowie entsprechende Schutzzubereitungen, die für diese Zweck eingesetzt werden können.

### BESCHREIBUNG DER ERFINDUNG

Gegenstand der Erfindung ist eine Reagensmischungen, enthaltend
(a) eine PCR-Grundmischung, und
(b) einen kohlenhydrathaltigen Schutzstoff, umfassend oder bestehend aus
   (b1) mindestens einen Stabilisator aus der Gruppe der Disaccharide und/oder Trisaccharide,
   (b2) mindestens einen Gerüststoff aus der Gruppe der Dextrane, Dextrine oder Proteine, sowie gegebenenfalls
   (b3) mindestens einem oberflächenaktiven Stoff.
Vorzugsweise liegt die Reagensmischung als trockenes Pulver, insbesondere als lyophilisiertes (gefriergetrocknetes) Pulver vor, das eine Restfeuchte von weniger als 2 Gew.-%, vorzugsweise weniger als 1 Gew.-% und insbesondere von etwa 0,1 bis 0,5 Gew.-% aufweist.

Überraschenderweise wurde gefunden, dass die kohlenhydrathaltigen Zubereitungen, die die Komponente (b) bilden, in vorzüglicher Weise geeignet sind, PCR-Grundmischungen, die empfindliche Zellen, Enzyme und Nucleotide enthalten, unter den Bedingungen der Gefriertrocknung vor Schädigungen zu schützen.

### REAGENSMISCHUNGEN

Die Reagensmischungen der vorliegenden Erfindung enthalten als ersten Bestandteil eine so genannte PCR-Grundmischung. Hierunter sind dem Fachmann Zubereitungen bekannt, die mindestens einen der Stoffe umfasst, die ausgewählt sind aus der Gruppe, die gebildet wird von Polymerasen, DNA-Glycosylasen, Nukleosidtriphosphate (NTP) und Oligonucleotiden. Grundsätzlich können in dieser Grundmischung alle Komponenten enthalten sein, die erforderlich sind, um eine definierte PCR durchzuführen. Dazu gehören des Weiteren beispielsweise Elektrolyte, Puffer, BSA (Bovine Serum Albumine), Farbstoffe, Wasser und dergleichen.

### KOHLENHYDRATHALTIGE SCHUTZSTOFFE

### Stabilisatoren

Als erste Komponente enthalten die kohlenhydrathaltigen Schutzstoffe Stabilisatoren, welche vorzugsweise ausgewählt sind aus der Gruppe, die gebildet wird von Saccharose, Raffinose und Trehalose.

Saccharose stellt ein Disaccharid aus α-D-Glucose und β-D-Fructose dar.

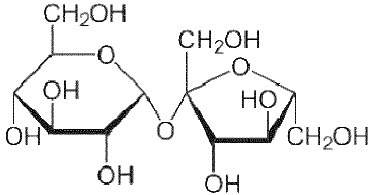

Bei der Raffinose handelt es sich um ein Trisaccharid bestehend aus Galactose, Glucose und Fructose:

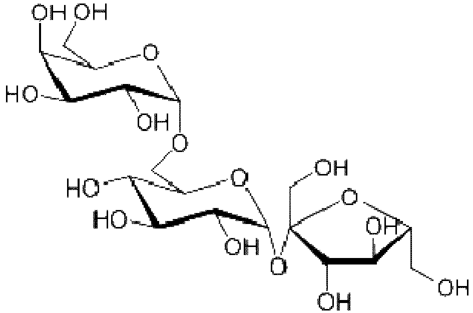

Die Trehalose stellt wieder ein Disaccharid dar, genauer um 1-α-Glucopyranosyl-1-α-Glucopyranosid:

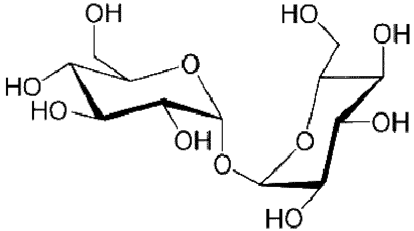

### Gerüststoffe

Als zweite Komponente enthalten die Schutzstoffe Gerüststoffe, die vorzugsweise ausgewählt sind aus der Gruppe, die gebildet wird von Dextrinen, Maltodextrinen und Gelatine.

Dextrine, zu denen Maltodextrine eine Untergruppe bilden, stellen Stärkeabbauprodukte dar, die von ihrer Molekülgröße her zwischen Oligosacchariden und Stärke liegen. Üblicherweise kommen sie in Form von weißem bzw. hellgelbem Pulver vor. Sie werden hauptsächlich aus Weizen-, Kartoffel- und Maisstärke durch trockene Erhitzung (>150 °C) oder unter Säureeinwirkung gewonnen. In der Natur wird Dextrin zum Beispiel von *Bacterium macerans* erzeugt. Dextrine entstehen auch durch den enzymatischen Abbau von Stärke durch Amylase.

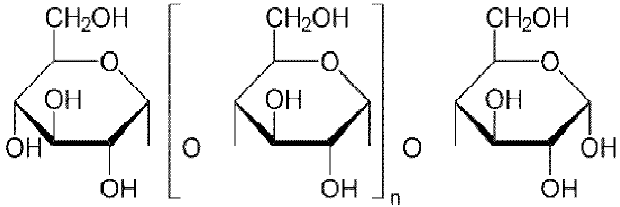

Bei der enzymatischen Spaltung von Stärke werden die Polymere in Mono-(Dextrose), Di- (Maltose) und Oligosaccharide gespalten. Die dabei gebildeten Produkte werden, je nach ihrem Abbaugrad (Dextrose-Äquivalent oder DE-Wert) als Maltodextrin bzw. Glucosesirup bezeichnet. Amylopektin und Amylose werden von der α-Amylase in Oligosaccharide gespalten, welche wiederum von β-Amylase in Maltose und schließlich in α-D-Glucose (auch Dextrose oder Traubenzucker genannt) mittels α-Glucosidase gespalten werden können. Unter den Maltodextrinen sind solche bevorzugt, die einen DE-Wert im bereich von 5 bis 20 aufweisen.

Als alternative Gerüststoffe kommen auch Proteine, speziell Gelatine in Frage. Hauptbestandteil der Gelatine ist dabei denaturiertes bzw. hydrolysiertes Kollagen, das aus dem Bindegewebe verschiedener Tierarten, vor allem Schweinen und Rindern, gewonnen wird.

### Oberflächenaktive Stoffe

Als optionalen Bestandteile können die kohlenhydrathaltigen Schutzstoffe oberflächenaktive Stoffe, d.h. anionische, nichtionische, kationische und/oder amphotere bzw. zwitterionische Tenside enthalten. Vorzugsweise kommen nichtionische Tenside zum Einsatz, die über ein hohes Emulgiervermögen verfügen. Hierzu zählen insbesondere:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:

**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di-und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Besonders bevorzugt sind Alkylphenolethoxlate, wie beispielsweise Triton X 100 (Octylphenol+10EO) oder alkoxylierte Sorbitanester, wie beispielsweise Tween 20

### (Sorbitanmonolaurat+20EO)

### Zubereitungen

Die erfindungsgemäßen Mischungen zeichnen sich dadurch aus, dass sie die Komponenten (a) und (b) im Gewichtsverhältnis von etwa 70:30 bis etwa 95:5 enthalten. Vorzugsweise beträgt das Mischungsverhältnis etwa 75:25 bis etwa 90:10 und insbesondere etwa 80:20 bis etwa 85:15.

Die Schutzstoffe, die die Gruppe (b) umfassen, können die Komponenten (b1), (b2) und (b3) im Gewichtsverhältnis 10:(1-3):(0-1, vorzugsweise im Gewichtsverhältnis 10: (1,5-2):(0,1-0,5) enthalten.

### KOHLENHYDRATHALTIGE ZUBEREITUNGEN

Ein weiterer Gegenstand betrifft kohlenhydrathaltige Zubereitungen, enthaltend oder bestehend aus
(b1) mindestens einen Stabilisator aus der Gruppe der Disaccharide und/oder Trisaccharide,
(b2) mindestens einen Gerüststoff aus der Gruppe der Dextrane, Dextrine oder Proteine, sowie gegebenenfalls
(b3) mindestens einem oberflächenaktiven Stoff,
die in besonderer Weise geeignet sind, die PCR-Grundmischungen gegen die schädlichen Einflüsse bei der Gefriertrocknung zu schützen.

Diese Zubereitungen enthalten vorzugsweise Stabilisatoren, die ausgewählt sind aus der Gruppe, die gebildet wird von Saccharose, Raffinose und Trehalose. Bezüglich der bevorzugten Ausführungsformen wird auf die Erläuterungen oben verwiesen, so dass sich eine Wiederholung erübrigt.

Die Zubereitungen enthalten vorzugsweise Gerüststoffe, die ausgewählt sind aus der Gruppe, die gebildet wird von Dextrane, , Maltodextrinen und Gelatine. Bezüglich der bevorzugten Ausführungsformen wird auf die Erläuterungen oben verwiesen, so dass sich eine Wiederholung erübrigt.

Die Zubereitungen schließlich als optionale Bestandteile oberflächenaktive Stoffe, bei denen es sich insbesondere um nicht-ionische Emulgatoren handelt. Bezüglich der bevorzugten Ausführungsformen wird auch hier auf die Erläuterungen oben verwiesen, so dass sich eine Wiederholung erübrigt.

In einer besonders bevorzugten Ausführungsform der vorliegenden Erfindung enthalten die Zubereitungen mindestens zwei Stabilisatoren, mindestens einen Gerüststoff und mindestens einen oberflächenaktiven Stoff enthalten. Bevorzugte Stabilisatorkombinationen sind hier Saccharose/Raffinose/Trehalose oder Raffinose/Trehalose, wobei die Komponenten vorzugsweise in annähernd gleichen Mengenverhältnissen zugegen sind.

### Weiter bevorzugt sind Zubereitungen die

(b1) etwa 70 bis etwa 99 Gew.-%, vorzugsweise etwa 75 bis etwa 95 Gew.-% und insbesondere etwa 80 bis etwa 90 Gew.-% Stabilisatoren,
(b2) etwa 1 bis etwa 30 Gew.-%, vorzugsweise etwa 5 bis etwa 25 Gew.-% und insbesondere etwa 10 bis etwa 20 Gew.-% Gerüststoffe sowie
(b3) 0 bis etwa 5 Gew.-%, vorzugsweise etwa 0,1 bis etwa 3 Gew.-% und insbesondere etwa 0,5 bis etwa 1 Gew.-% oberflächenaktive Stoffe
mit der Maßgabe enthalten, dass sich die Mengenangaben jeweils zu 100 Gew.-% ergänzen.

### GEWERBLICHE ANWENDBARKEIT

Ein weiterer Gegenstand der vorliegenden Erfindung betrifft ein Verfahren zur Herstellung einer stabilisierten Reagensmischung, bei dem man eine PCR-Grundmischung mit einer kohlenhydrathaltigen Zubereitung wie oben beschrieben versetzt und die Mischung dann gemeinsam einer Gefriertrocknung unterwirft.

Unter dem Begriff Gefriertrocknung oder Lyophilisierung versteht man ein Verfahren zur Trocknung lösungsmittelhaltiger, speziell wässriger Zubereitungen. Die Gefriertrocknung beruht auf dem physikalischen Prozess der Sublimation: Bei der Gefriertrocknung sublimieren die Eiskristalle ohne zwischenzeitliches Auftreten einer flüssigen Phase direkt in den gasförmigen Zustand. Das Endprodukt der Gefriertrocknung wird als Lyophilisat bezeichnet. Die Gefriertrocknungsanlage besteht aus zwei Kammern, die miteinander verbunden und durch ein Ventil verschließbar sind. Das zu trocknende Produkt steht auf einer beheiz- und kühlbaren Stellfläche (Horden) und wird zuerst bei Normaldruck tiefgefroren. In die zweite Kammer ist eine Rohrschlange (Kondensator) eingebaut, die von einer kalten Sole oder einem Kältemittel durchströmt wird. Im nächsten Schritt der Primärtrocknung wird das im Gut enthaltene Wasser sublimiert. Das beruht auf dem Prinzip, dass Wasser auch im gefrorenen Zustand einen ausreichend hohen Dampfdruck hat, um direkt vom gefrorenen in den gasförmigen Aggregatzustand überzugehen. Dazu wird ein Vakuum angelegt.

Bei der Sublimation wird Energie aufgenommen. Weil thermische Energie aus der Umgebungstemperatur bezogen wird, würde sich die Temperatur in der Trocknungskammer im Laufe des Prozesses senken; um die Temperatur konstant zu halten, wird der Kammer daher so viel Wärme zugefügt, wie vom Wasser als Sublimationsenergie aufgenommen wird. Im Verlauf des Trocknungsprozesses besteht die Atmosphäre in den Kammern fast ausschließlich aus Wasserdampf, der sich als Eis auf den kalten Rohrschlangen des Kondensators niederschlägt. Im weiteren Prozess folgt die Sekundärtrocknung, bei der durch weiteres Erwärmen stärker gebundenes Wasser aus dem Produkt entfernt wird.

Für die Kühlung wird entweder ein Kälteträger (beispielsweise Silikonöl) eingesetzt, oder es erfolgt die direkte Beaufschlagung mit einem Kältemittel (typisch: Stickstoff oder CO₂). Die Temperaturen des Kondensators liegen typischerweise bei -60 bis -80 °C. In großtechnischen Anlagen werden oft Absorptionskältemaschinen mit dem Kältemittel Ammoniak verwendet. Der Kondensator kann durch Wasserdampf abgetaut werden, nachdem die Verbindungsklappe geschlossen wurde. Die Prozessführung kann als Batchverfahren(chargenweise Trocknung), aber auch kontinuierlich oder semi-kontinuierlich erfolgen.

Dabei setzt man die PCR-Grundmischung und die kohlenhydrathaltige Zubereitung vorzugsweise im Gewichtsverhältnis von etwa 70:30 bis etwa 95:5, insbesondere etwa 80:20 bis etwa 90:10 ein.

Ein letzter Gegenstand der Erfindung betrifft die Verwendung der kohlenhydrathaltigen Zubereitungen wie oben beschrieben als Schutzstoffe bei der Gefriertrocknung von Reagensmischungen, speziell solchen die für die PCR eingesetzt werden, und empfindliche Zellen, Enzyme und Nucleotidsequenzen enthalten.

### ALLGEMEINE HERSTELLVORSCHRIFT

Die Abfüllung des der Reagensmischungen erfolgte in einer 12 x 8ter Streifenmatte zu je 20 µl je PCR Tube. Anschließend wurde die Mischung bei -25°C tiefgefroren. Die gefrorenen Produkte wurden dann in das Gerät zur Gefriertrocknung überführt und lyophilisiert. Die Gefriertrocknung erfolgt unter Vakuum mit einer stufenweisen Temperaturerhöhung von -45 bis 25°C.

Die auf diese Weise lyophilisierten Reagensmischungen konnten dann z.B. für eine PCR oder RT- PCR Reaktion eingesetzt werden, indem man die Mischungen beispielsweise mit 25µl Template (DNA oder RNA) resuspendiert. Anschließend erfolgte die Reaktion in einem entsprechenden PCR Gerät.

Um die jeweiligen Reagensmischungen für die Gefriertrocknung tauglich zu machen, wurden alle Grundkomponenten mit jeweils spezifischen Schutzkomponenten bestehend aus mindestens einem Stabilisator, mindestens einem Gerüststoff sowie mindestens einem Tensid stabilisiert. In der folgenden Tabelle sind beispielhaft Mengenangaben wiedergegeben. Die Angaben sind dabei so zu verstehen, dass die Mengen an Schutzkomponenten (bestehend aus den drei Einzelkomponenten) im Bereich von etwa 5 bis etwa 10 Gew.-% liegen und sich mit der Menge an PCR-Grundmischung zu 100 Gew.-% ergänzen. Die Menge an Stabilisatoren beträgt jeweils etwa 4 bis 10 Gew.-%, an Gerüststoffen etwa 0,5 bis etwa 1 Gew.-% und die Menge an Tensiden etwa 0,01 bi etwa 1 Gew.-%.

**Tabelle 1**

| **Basiszusammensetzung** (Mengenangaben als Gew.-%) | | |
|---|---|---|
| **Grundkomponenten** | **Reagenz** | **Menge** |
| PCR Grundmix | Polymerase | Ad 100 |
| | Uracil-DNA Glycosylase | |
| | PCR Puffer | |
| | Wasser | |
| | MgCl2 | |
| | dNTP's | |
| | Oligonukleotide | |
| | BSA | |
| | Chinolingelb | |

## Patentansprüche

1. Reagensmischungen, enthaltend
(a) eine PCR-Grundmischung, und
(b) einen kohlenhydrathaltigen Schutzstoff, umfassend oder bestehend aus
(b1) mindestens einen Stabilisator aus der Gruppe der Disaccharide und/oder Trisaccharide,
(b2) mindestens einen Gerüststoff aus der Gruppe der Dextrine oder Proteine, sowie gegebenenfalls
(b3) mindestens einem oberflächenaktiven Stoff.

2. Mischungen nach Anspruch 1, **dadurch gekennzeichnet, dass** sie PCR-Grundmischungen enthalten, die mindestens einen der Stoffe umfassen, die ausgewählt sind aus der Gruppe, die gebildet wird von Polymerasen, DNA-Glycosylasen, NTP und Oligonucleotiden.

3. Mischungen nach den Ansprüchen 1 und/oder 2, **dadurch gekennzeichnet, dass** sie Stabilisatoren enthalten, die ausgewählt sind aus der Gruppe, die gebildet wird von Saccharose, Raffinose und Trehalose.

4. Mischungen nach mindestens einem der Ansprüche 1 bis 3, **dadurch gekennzeichnet, dass** sie Gerüststoffe enthalten, die ausgewählt sind aus der Gruppe, die gebildet wird von Dextrane, Maltodextrinen und Gelatine.

5. Mischungen nach mindestens einem der Ansprüche 1 bis 4, **dadurch gekennzeichnet sind, dass** sie als oberflächenaktiven Stoffe nichtionische Emulgatoren enthalten.

6. Mischungen nach mindestens einem der Ansprüche 1 bis 5, **dadurch gekennzeichnet, dass** sie die Komponenten (a) und (b) im Gewichtsverhältnis von etwa 70:30 bis etwa 95:5 enthalten.

7. Mischungen nach mindestens einem der Ansprüche 1 bis 6, **dadurch gekennzeichnet, dass** die Schutzstoffe die Komponenten (b1), (b2) und (b3) im Gewichtsverhältnis 10:(1-3):(0-1) enthalten.

8. Kohlenhydrathaltige Zubereitungen, enthaltend oder bestehend aus
(b1) mindestens einen Stabilisator aus der Gruppe der Disaccharide und/oder Trisaccharide,
(b2) mindestens einen Gerüststoff aus der Gruppe der Dextrine oder Proteine, sowie gegebenenfalls
(b3) mindestens einem oberflächenaktiven Stoff.

9. Zubereitungen nach Anspruch 8, **dadurch gekennzeichnet, dass** sie Stabilisatoren enthalten, die ausgewählt sind aus der Gruppe, die gebildet wird von Saccharose, Raffinose und Trehalose.

10. Zubereitungen nach den Ansprüchen 8 und/oder 9, **dadurch gekennzeichnet, dass** sie Gerüststoffe enthalten, die ausgewählt sind aus der Gruppe, die gebildet wird von Dextrane, , Maltodextrinen und Gelatine.

11. Zubereitungen nach mindestens einem der Ansprüche 8 bis 10, **dadurch gekennzeichnet, dass** sie mindestens zwei Stabilisatoren, mindestens einen Gerüststoff und mindestens einen oberflächenaktiven Stoff enthalten.

12. Zubereitungen nach mindestens einem der Ansprüche 8 bis 11, **dadurch gekennzeichnet, dass** sie
(b1) etwa 70 bis etwa 99 Gew.-% Stabilisatoren,
(b2) etwa 1 bis etwa 30 Gew.-% Gerüststoffe sowie
(b3) 0 bis etwa 5 Gew.-% oberflächenaktive Stoffe
mit der Maßgabe enthalten, dass sich die Mengenangaben jeweils zu 100 Gew.-% ergänzen.

13. Verfahren zur Herstellung einer stabilisierten Reagensmischung, bei dem man eine PCR-Grundmischung mit einer kohlenhydrathaltigen Zubereitung nach Anspruch 8 versetzt und die Mischung dann einer Gefriertrocknung unterwirft.

14. Verfahren nach Anspruch 13, **dadurch gekennzeichnet, dass** man die PCR-Grundmischung und die kohlenhydrathaltige Zubereitung im Gewichtsverhältnis von etwa 70:30 bis etwa 95:5 einsetzt.

15. Verwendung der kohlenhydrathaltigen Zubereitungen nach Anspruch 8 als Schutzstoffe bei der Gefriertrocknung von Reagensmischungen des Anspruchs 1.
